# EUROPEAN PATENT SPECIFICATION

(11) **EP 2 986 176 B1**
(45) Date of publication and mention of the grant of the patent: **28.03.2018**
(21) Application number: 14728318.8
(22) Date of filing: 18.04.2014
(51) Int. Cl.: A41H 1/02, A61B 5/107

(54) **DYNAMOMETRIC TAPE METER FOR APPLICATION IN THE TREATMENT OF LYMPHEDEMA**
DYNAMOMETRISCHE BANDMESSVORRICHTUNG ZUR VERWENDUNG BEI DER BEHANDLUNG VON LYMPHÖDEMEN
RUBAN-MÈTRE DYNAMOMÈTRIQUE DESTINÉ A ÊTRE APPLIQUER DANS LE TRAITEMENT DE LYMPHOÉDÈME

(30) Priority: 18.04.2013 IT MI20130632
(43) Date of publication of application: 24.02.2016
(73) Proprietor: Farina, Giovanni, 20151 Milano (IT)
(72) Inventor: Farina, Giovanni, 20151 Milano (IT)
(74) Representative: Croce, Valeria
(86) International application number: PCT/IB2014/060837
(87) International publication number: WO 2014/170877

(56) References cited:
- DE-B- 1 052 696
- R. LA FORGE: "Office-based Anthropometric Measures", PUBLIC HEALTH SERVICE, 1996, pages 1-6, XP002717958,
- K. G. BRODOVICZ ET AL: "Reliability and Feasibility of Methods to Quantitatively Assess Peripheral Edema", CLINICAL MEDICINE & RESEARCH, vol. 7, no. 1-2, 1 June 2009 (2009-06-01), pages 21-31, XP055079019, ISSN: 1539-4182, DOI: 10.3121/cmr.2009.819

## Description

It is the object of the present invention a dynamometric tape meter which finds use in the medical field, and particularly in the field of the treatment and diagnosis of lymphedematous limbs.

Lymphedema is a chronic disease condition with an evolution trend, characterized by a volumetric thickening of the limbs and tissue fibrosis.
All the major international authors agree that a combination of several treatments is necessary, referred to as the "ComplexPhysicalTherapy" (Casley-Smith, Leduc, Földi, VodderConsensus Conference - New York, 1998).
The "ComplexPhysicalTherapy" is based on the integration of multiple therapies, such as: skin cleansing, manual lymphatic drainage, multilayer bandaging, decongestive exercises on the bandaged limb, and so on, each of which concurs to a different extent to the achievement of the clinical result.
In order to dimension with the maximum possible precision the lymphedematous limb, beside monitoring the trend during the therapy and to quantify the result achieved by the treatment, it is necessary to carry out a volumetric measurement of the limbs.
Water volumetry is the gold standard, and it is the optimal study to measure the limb volume.
The limb is introduced up to a preset level into a container that has previously been filled with water, and the water displacement volume is measured.
On the contrary, the water volumetric method exhibits a series of elements such as to make this technique not very usable as a routine practice (consider the necessity to provide the container, which is not always easy and feasible in the hospital setting), in addition to not provide any indications on the edema spatial distribution.
For these reasons, most of the international lymphology centers adopt an indirect volumetric methodology, comprising: technique at preset heights, technique with identification of bony landmarks, mixed technique (preset heights and bony landmarks), segment technique, etc..
Through the use of the indirect volumetry, it is possible to obtain in a sufficiently precise manner the limb volume by applying special formulae for calculating the geometric solids, to which the various segments of the limb are assimilated.
Once the measurement point of the skin tissue have been identified, it is necessary to detect the circumference by using a tape meter, and to determine the volumes by suitable mathematical models.

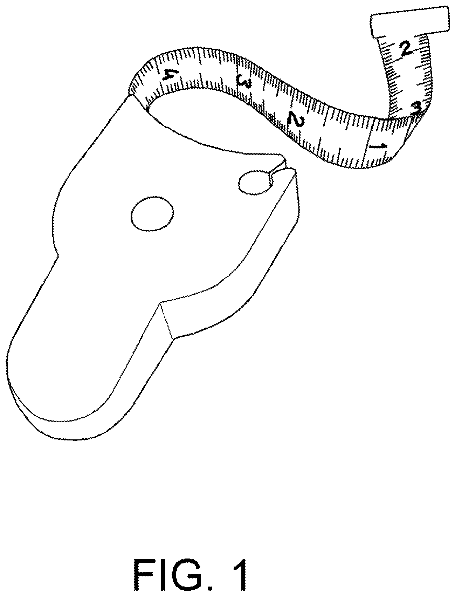

Several types of tape meter are in the market, but no one of them has a system for controlling the bidirectional tension such as to reduce the intra- and inter-operatory variability in detecting the circumference.
The only tool currently commercially available, capable of measuring a given point with the same tension, is a tape meter with an anatomical support provided with a spring return (illustrated in Fig. 1). The so-called "Gulick tape measure" and instructions for using the device are described in "Office-based Anthropometric Measures" (by R. La Forge, published 1996 by the U.S. Department of Health and Human Services). However, such a tool can not be disinfected in each of its component, as required by hospital procedures for the protection of both the operator and the patient, the latter being, under such physiologic status, particularly exposed to the infection risk.
Furthermore, such tool may oversize the circumferential measurement also due to a predetermined difficulty in the positioning thereof, and it is not able to measure all the anatomical districts (hand, fingers, etc.).
It shall be also stated that the patient affected by a lymphedema has a fibrosclerotic alteration of the tissues, which has to be assessed in order to classify the disease stage from the fibrosclerotic viewpoint, in addition to the assessment from the algorithmic viewpoint which procedure shall be used for the compressive therapy.

Currently, the only one commercially available equipment that is capable of carrying out this type of detection is the tonometer.
However, such a tool, while being extremely accurate and repeatable, cannot be used on the districts of the limb other than those intended, since the tool cannot be positioned. Furthermore, it is very expensive ("Valutazione strumentale nel linfedema: la tonometria tissutale", Claudio Puglisi).

Therefore, the purpose of the present invention is to provide a simple, not very expensive tool, allowing carrying out precise and reproducible measurements of both the real circumference of a limb and the tissue consistency.

The present invention will be described herein below with reference to the appended figures, in which:
Fig. 1 shows a device known in the art;
Fig. 2 shows the device of the invention;
Figs. 2A and 2B show details of the device of Fig. 2;
Fig. 3 shows the device of Fig. 2 in use;
Figs. 4 and 5 show examples of mathematical models for the indirect volumetric measurement of the limbs.

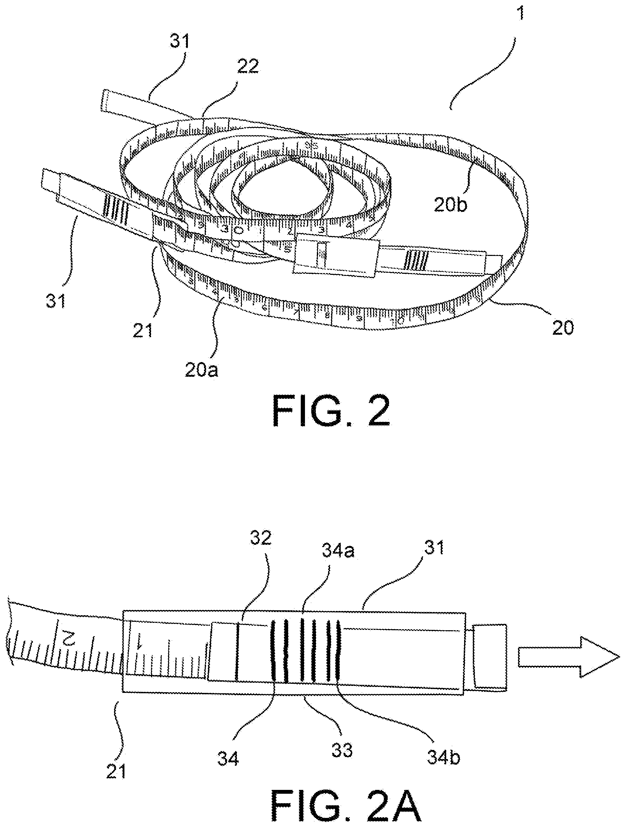

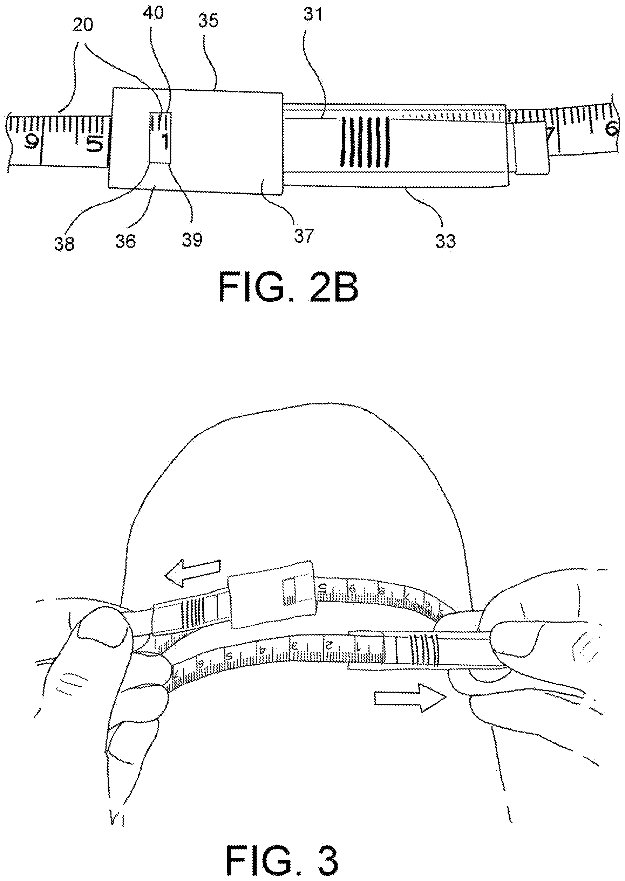

With reference to Fig. 2, a device according to the invention is generally indicated with 1.
Particularly, the device 1 comprises measurement means 20 represented for example by a tape having a main extension (or length) dimension.
The tape meter comprises a graduated scale, preferably on one or both the faces 20a,20b, preferably in centimeters, according to the decimal system, and/or in inches, according to the needs.
In order to adapt to the limb shapes and curves, the tape is made of a flexible material.
In a preferred aspect, such tape can be represented by a tape meter of the type commonly commercially available, which will be referred to herein below.
In a still more preferred aspect of the invention, it can be provided for said material to be also anallergic, so as not to cause reactions upon skin contact. Furthermore, such material is preferably washable and optionally also sterilizable, so that it can be safely reused while excluding the risk of possible infections.

The device 1 of the invention further comprises means 31 for tensioning the tape meter.
Such means are configured to apply a bidirectional tensioning force to the tape when they are pulled away from the tape itself.
In other words, it is the force which, when applied to the tensioning means in a direction opposite the tape ends (as indicated by the arrow of Fig. 3), allows obtaining its full distension and adhesion to the limb surface, without folds or distortions.
In a preferred aspect, such tensioning means 31 comprise resilient members, for example, comprising members made of an elastomeric material, such as rubber, or a spring, or another equivalent member.
The elastic constant of the tensioning means 31 will be selected so as to impart a predetermined constrictive force on the patient's limb, and repeatable between a measurement and another one.
In another aspect of the invention, the tensioning means 31 can be represented by a dynamometer, also of the digital type.
Therefore, a correct measurement of the limb can be advantageously achieved, also avoiding an undue deepening.
In an aspect of the invention, the tensioning means 31 can be connected to the tape meter ends.
Alternatively, one or both the tensioning means can be slidably associated to the tape, as it will be described herein below.
In an aspect of the invention, the tensioning means 31 are both coupled to an end of the tape 20, and slidably coupled to the tape 20.

Furthermore, according to the present invention, the tensioning means 31 are associated with or comprise distension indication means 32 and control means 33.
In a preferred aspect, the indication means 32 are represented by one or more members integral to the tensioning means 31.
Particularly, they can be represented by a notch, a relief, or a reference line arranged in a suitable position on the tensioning means 31.
Therefore, when the tensioning means 31 are subjected to said tensioning force, the indication means 32 move away from or towards the corresponding ends 21, 22 of the tape 20.

As regards the control means 33, these are configured to verify the displacement of the indication means 32 from the rest position, i.e., when no tensioning force is applied, to the relative position taken following the application of the tensioning force. Advantageously, the indication means 32, together with the control means 33, allow verifying the tensioning degree, in other words, the force applied to the tape via the tensioning means 31, hence also the constrictive force applied to the limb.
This is obtained by aligning the indication means 32 with the control means 33.
In a preferred aspect of the invention, said control means 33 comprise one or more control members 34, 34a, 34b, which therefore can be in association with each tensioning means 31.
A different tensioning force corresponds to each control member 34, 34a, 34b.
Since the tensioning means 31 are made of a resilient material, a greater applied force will correspond to a greater displacement of the indication member 32 with respect to its rest position (i.e., the position taken in the absence of an applied force) and with respect to the control means 33.
In a preferred embodiment, the control members 34, 34a, 34b can be composed, for example, by a notch, a relief, or a reference line.
For the purposes of the present invention, the control members 34, 34a, 34b are stationary, hence their position with respect to the point of the tape to which they are associated is stationary (as well as they are stationary with respect to the position of the indication members 32 in the rest situation) both under rest conditions, and under a tensioning force application condition.
Therefore, they are preferably configured so that their position is visible independently of the tensioning means 31 and the indication members 32 position.
For example, in a particularly preferred embodiment, the control means 33 are composed of a transparent material member, for example, transparent plastic, which overlaps the tensioning means and on which one or more control members 34, 34a, 34b are indicated, specifically control lines (as shown, for example, in Fig. 2B). Advantageously, therefore, through the transparent member it is possible to easily verify the displacement of the tensioning indication members 32 following the application of the force.
It shall be also noticed that, by virtue of control means 33, it is possible to verify that the tensioning force applied to the tape meter through both tensioning means 31 is always the same; in the same manner, it is possible to verify time after time the repeatability of the performed measurement.

Alternatively to the embodiment where the device of the invention comprises tensioning means 31 and control means 33 at each end 21,22 of the tape meter, it is possible to provide that a tensioning means 31/control means 33 pair is coupled to an end of the tape 20, while a second tensioning means 31/control means 33 pair is slidably associated to the same tape 20.

Particularly, it can be provided for the tensioning 31 and control 32 means to be associated with sliding means 35 (represented in Fig. 2B), which are configured to move along the tape 20, i.e., along its main extension dimension.
Such sliding means 35 can be represented, for example, by a member, for example, made of plastic or composed of a thin sheet of a metal, or paper, or paperboard, or equivalent materials.

In more detail, said sliding member 35 comprises a coupling portion 36 for sliding coupling to the tape 20 and a constraint portion 37 to the tensioning means 31 and the control means 33.
As regards the coupling portion 36, this may comprise, for example, means for adjusting the position of the sliding member 35 with respect to the tape, configured to pass the tape 20 therein, and such as to create a friction portion, in which a friction force between the sliding member 35 and the tape 20 is created, which is greater than the tensioning force to be applied to the tensioning means 31 in order to obtain the displacement thereof.
Said adjusting means can comprise, for example, one or more through slits 38,39, so that the tape 20 passes through one of them, or both, with a predetermined friction.
Preferably, at least two slits 38 and 39, are provided for, which are mutually spaced apart by a friction portion 40, the first 38 of which allows the passage of the tape 20 from the first slit 20a to the second face 20b, and from the latter 20b through the second slit 39 again to the first face 20a, respectively, so that a frictional sliding of the tape 20 in the sliding member 35 is created.
In this manner, a coupling between the tape 20 and the sliding member 35 is formed, which, by virtue of the sliding friction that is created, is such as to advantageously keep the sliding member 35 in a given position along the tape 20.
In an embodiment, such friction force may increase as the operator, by applying a predetermined force on the tensioning means 31 connected to the sliding member 35, produces therein, particularly at the slits 38,39, a deformation which, while being limited, helps to further hold the tape 20, thus preventing the sliding thereof. Therefore, the tensioning force applied by the operator to the tensioning means 31 will be not sufficient to produce the sliding member 35 sliding.
In fact, in order to alter the position thereof along the tape, it is necessary to force the passage of the tape 20, first pushing it through one of the two slits 38,39 and subsequently by pulling it through the other one 38,39.
In other embodiments, the means for adjusting the position of the sliding member 35 on the tape 20 can be obtained by shaping the same sliding member 35 as a slid or a sleeve and by arranging it astride of the tape 20.

According to an object of the present invention, the device 1 above described is used to measure the dimension of the limbs in a subject, particularly by measuring the limb transversal circumference (real circumference) in a predetermined point.

Particularly, for the measurement in a predetermined (imaginary) section of the limb, the operator keeps one of the ends 21,22 of the tape mater 20 stationary in one point, and transversally fully surrounds the limb according to such circumference (as shown in Fig. 3).
Subsequently, the operator applies a predetermined tensioning force to the tape with the tensioning means 31 (of course, the traction will be in the opposite direction the one with respect to the other one, as illustrated in Fig. 3).
In this manner, the tape meter 20 elongates and completely adheres to the limb surface according to such circumference, without folds or distortions, or deepening on the skin tissue.
The operator can verify having applied the same force at both ends 21,22 by assessing the position of each indication member 32 with respect to the control element (s) 34,34a,34b (this by virtue of the fact that the tensioning means 31 are made of the same material, or anyhow by a material characterized by the same elastic constant).

Therefore, under these conditions, while keeping the tensioning force constant, the measurement of the real circumference is therefore easily and clearly visible by the operator taking into account the zero position on the meter graduated scale.

The real circumference value may be therefore used to calculate the limb volume by means of suitable mathematical models, which simplify a limb shape by dividing it into a predetermined number of solids, so that the overall limb volume is approximately given by the sum of the volumes of such individual solids.

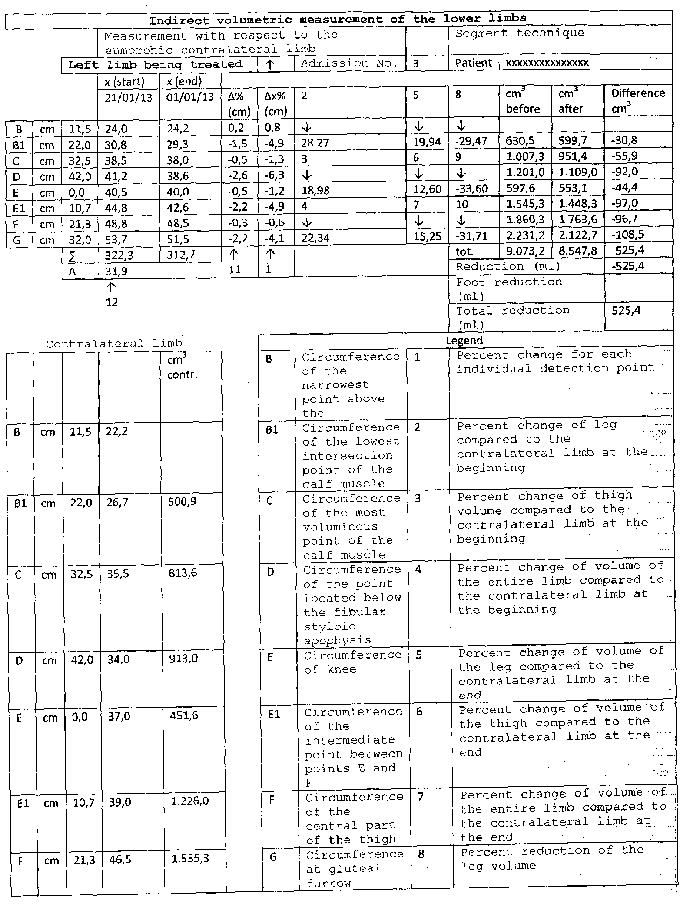

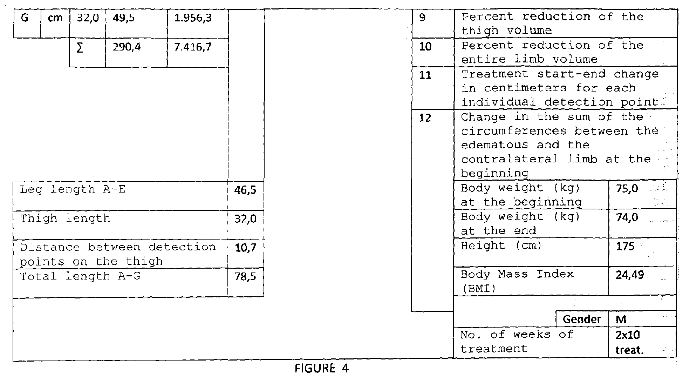

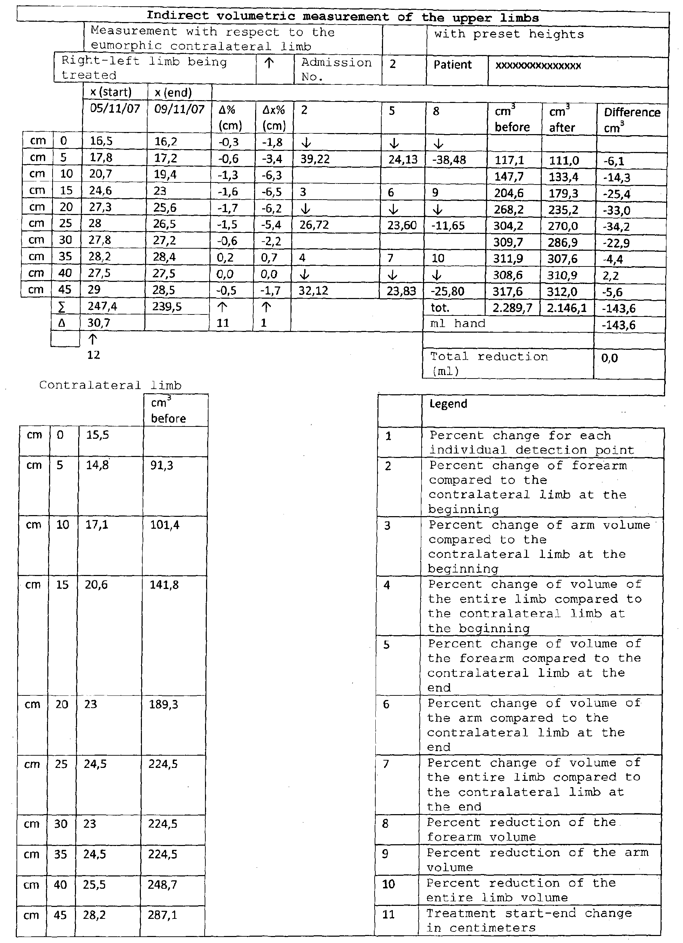

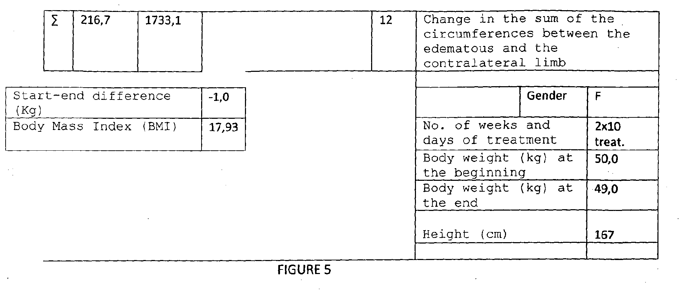

Figs. 4 and 5 show some examples of mathematical models for the indirect volumetric measurement of the lower and upper limbs by a comparison with the contralateral limb.

According to a further aspect of the present invention, the described device 1 is also useful to provide information about the state of the tissues of the measured limb (the so-called "tissue consistency") and, for example, to assess its alteration from a fibrosclerotic viewpoint.
Particularly, it is information allowing to obtain *ad interim* parameters and, therefore, they do not *per se* constitute a diagnosis; in fact, they require in any event the intellectual contribution of one skilled in the art.
Such information relates to the consistency and compressibility of the tissues.
By consistency it is meant the resistance degree opposed by the limb tissue to a mechanical compression caused by the tension applied through the tape, and it is the result of possible evolutions of the edematous tissue from a fibrosclerotic viewpoint ("Valutazione strumentale nel linfedema: la tenometria tissutale", Dr. Claudio Puglisi).
As regards the compressibility, instead, it comprises searching for the fovea, i.e., of that depression, following a compression by fingers, caused by the displacement of the interstitial fluid about the compression point.

From a practical viewpoint, the operator, after applying such a tensioning force to produce the complete distension and adhesion of the tape to the limb (which circumstance allows measuring the real circumference) as described above, can increase the tensioning force exerted onto the tensioning means 31, thus producing a deepening force.

In this manner, it produces a higher compression, which causes a (transitory) deformation of the limb.

Particularly, such a deformation will cause a deepening of about 1-3%, preferably of about e 2%, i.e., it will cause a decrease of about 1-3%, and preferably of about 2% of the circumference with respect to the real circumference.

The arrangement of two or more control members 34, 34a, 34b allows applying tensioning forces, hence constrictive forces on the limb, which are variable and known, by virtue of the possibility that the operator verifies the extent of the force applied onto the tensioning means 31.

In a preferred aspect of the invention, the deepening step is obtained in a time period of about 3-6, and preferably about 3-5 seconds.
Given that, while keeping the applied force constant, the deformation depends on the status of the compressed tissues, the observation of the deformation extent and mode, obtained by applying tensioning forces having a different extent, the operator can get information useful to make a diagnosis of the patient's status and to determine the dressing to be applied to the limb, besides assessing possible improvements of the tissue fibrosis at the end of the therapeutic treatment. Particularly, to this aim, the method for measuring the tissue consistency and the fibrosclerotic alteration of the tissues can be repeated multiple times during a therapy, so that its efficiency can be assessed.
Furthermore, by carrying out the measurements and the assessments on the corresponding contralateral limb, the operator can get very useful information to understand the treatment trend and efficiency.

As regards the position of the control members 34,34a,34b, these can be arbitrarily set by the operator, or arranged following preliminary tensioning and pre-calibration operations, respectively, by which it is possible to verify which is the intensity of the force applied to the tensioning means necessary to achieve the full distension of the tape 20 without folds or distortions, and to achieve a predetermined deformation of the tissues.
This operation can be carried out, for example, by drawing a sign (such as a line) on the tensioning means when these are in the rest position (i.e., when they are not subjected to any force) and subsequently by locating the device of the invention in a vertical position and connecting a mass, for example, of 25 g or 200 g or 300 g to the tensioning means.
At this point, the operator may identify the position of the control means at the position taken by the indication members when a force of 25g, 50g, 75g, etc., respectively, has been applied, until reaching, for example, a maximum force of 300 g.
These standard values are currently recognized as standard tension values, but further possible clinical studies may establish different, more suitable values in order to determine:
- the circumference that can be defined as real (in which case the full extension of the tape and an almost null deepening in the skin tissue occur);
- tissue consistency;
- tissue compressibility.

From the description set forth above of the device of the invention, those skilled in the art, hence the physician or physiotherapist, will be able to understand the various advantages offered compared to the already known devices.
First of all, the device is very simple to be used, not bulky and light-weighted, therefore it is absolutely portable.
Furthermore, it allows not only an accurate and reproducible measurement, which therefore is not affected or least affected by the "operator factor", of the limb volume, but it also allows assessing the edema spatial distribution both from a supine position and an orthostatic position.
A further and absolutely not negligible advantage is illustrated by the possibility of having indications about the possible fibrosclerotic alteration of the tissues.
By virtue of this, useful information will be able to be collected, to classify the disease stage from the fibrosclerotic viewpoint, thereby determining, by means of suitable tables and mathematical models, which compressive therapy procedure is the most suitable in order to improve the symptoms.
Furthermore, data and useful information will be able to be collected to be able to follow the disease evolution upon time, along with possible improvements, and to accordingly adapt the medical treatment.
Those skilled in the art will also be able to understand the usefulness of the device of the present invention also in different working fields.
For example, vascular surgeons, physiatrists, orthopaedists, dermatologists may use the device described.
Also posturologists and physiotherapists will find it useful, since it allows to objectively assess the possible differences at the gastrocnemius and the quadriceps due to an erroneous load distribution. Orthopaedists, in turn, will find it useful in order to assess the suitability of elastic supports and to manufacture them in a customized manner.
Furthermore, the device of the invention may find use in the dietary and dietary/cosmetic fields, for example, to provide a higher scientific validity to the therapies used in treating panniculopathy.
In this manner, the dietitian or the aesthetic surgeon can follow the results and developments of a patient's weight loss schedule.
Also in the tailor field, a more correct measurement can be achieved by virtue of the device described in the present application.
To the embodiments described above of the dynamometric tape meter, those skilled in the art, in order to meet contingent, specific needs, will be able to make a number of additions, modifications, or replacements of members with other functionally equivalent ones, without however departing from the scope of the appended claims. Each of the characteristics described as belonging to a particular embodiment can be implemented independently of the other embodiments described.

## Claims

1. A device (1) for measuring the circumference of a limb, comprising a tape meter (20) having two faces (20a,20b) and comprising a graduated scale and two ends (21,22), tensioning means (31) to apply a tensioning force (31) to said tape meter (20) capable of producing the fully distension thereof, wherein the tensioning means (31) comprise indication means (32) of said tensioning force (31) when subjected to said tensioning force, and control means (33) functionally coupled to said tensioning means (31), said control means (33) comprising control members (34,34a,34b) being able to cooperate with said indication means (32) to verify the displacement of the indication means (32) following the application of said tensioning force, **characterised in that** said tensioning means (31) are coupled to both ends (21,22) of said tape meter (20).

2. The device (1) according to the preceding claim, wherein said tensioning means (31) are slidably coupled to said tape (20).

3. The device (1) according to any one of the preceding claims, comprising tensioning means (31) coupled to one of the ends of said tape (20), and tensioning means (31) slidably coupled to said tape (20).

4. The device (1) according to any one of the preceding claims, wherein said tensioning means (31) and said control means (33) are mutually coupled.

5. The device (1) according to any of the claims 2 to 4, wherein said sliding coupling is obtained by sliding means (35) associated with said tape meter (20), said sliding means (35) being coupled to the tensioning means (32).

6. The device (1) according to the preceding claim, wherein said tensioning means (31) coupled to said sliding means (35) are further coupled to said control means (33).

7. The device (1) according to claim 5 or 6, wherein said sliding means (35) comprise adjusting means configured to obtain a sliding coupling with said tape meter (20).

8. The device (1) according to the preceding claim, wherein said adjusting means comprise at least a through slit (38,39) through which the tape (20) is passed, and defining a friction sliding portion (40) with said tape (20).

9. The device (1) according to any of the claims 5 to 8, wherein said adjusting means comprise two through slits (38,39) defining therebetween a friction sliding portion (40) with the tape (20).

10. The device (1) according to the preceding claim, wherein said sliding coupling is obtained by passing said tape meter (20) through said first slit (38) from the first face (20a) to the second face (20b) of the tape (20) and through said second slit (39) from the second face (20b) to the first face (20a) of the tape (20) astride of the friction sliding portion (40).

11. The device (1) according to any one of the preceding claims, wherein said tensioning means (32) are resilient means.

12. The device (1) according to the preceding claim, wherein said resilient means are represented by a helical spring or are made of a deformable material selected from elastomeric materials.

13. The device (1) according to any one of the preceding claims, wherein said control members (34,34a,34b) are stationary with respect to the position of the indication members (32) in the case where no tensioning force is applied to said tensioning means (32).

14. A method for measuring the real circumference of a limb in a predetermined point, comprising the use of the device (1) according to any of the claims 1 to 13, comprising the steps of:
1) applying a tensioning force to the tape meter (20) of the device (1), which tape meter (20) surrounds transversally a limb according to the circumference in said point, through the tensioning means (31), such as to produce the full distension of the tape (20) and the full adhesion to the limb surface;
2) while the tensioning force of step 1) is kept constant, verifying the indication of the circumference in said point on the graduated scale.

15. A method for measuring the tissue consistency in a point of a limb and for collecting information useful for assessing the fibrosclerotic alteration of the tissues of said limb, comprising the use of the device (1) according to any of the claims 1 to 13, comprising the steps of:
1) applying a tensioning force to the tape meter (20), which tape meter (20) surrounds transversally a limb according to the circumference in said point, through the tensioning means (31), such as to produce the full distension of the tape (20) and the full adhesion to the limb surface;
2) applying a deepening force through the tensioning means (31), such as to produce a deepening of the tape meter (20) onto the skin tissue;
3) while the deepening force of step 2) is kept constant, verifying the processed indication of the limb circumference in said point on the graduated scale and verifying the extent of the force of deepening applied by virtue of the verification of the position of the control means (33).

## Patentansprüche

1. Vorrichtung (1) zum Messen des Umfangs einer Extremität, umfassend ein Maßband (20) mit zwei Flächen (20a, 20b) und umfassend eine abgestufte Skala und zwei Enden (21, 22), Spannmittel (31), um eine Spannkraft (31) auf das Maßband (20) anzuwenden, welche in der Lage ist, die vollständige Dehnung davon zu erzeugen, wobei die Spannmittel (31) Anzeigemittel (32) der Spannkraft umfassen, wenn sie der Spannkraft unterzogen werden, und Regel-/Steuermittel (33), welche mit den Spannmitteln (31) funktionell gekoppelt sind, wobei die Regel-/Steuermittel (33) Regel-/Steuerelemente (34, 34a, 34b) umfassen, welche in der Lage sind, mit den Anzeigemitteln (32) zusammenzuwirken, um die Verlagerung der Anzeigemittel (32) auf die Anwendung der Spannkraft hin zu verifizieren,
**dadurch gekennzeichnet, dass** die Spannmittel (31) mit beiden Enden (21, 22) des Maßbands (20) gekoppelt sind.

2. Vorrichtung (1) nach dem vorhergehenden Anspruch, wobei die Spannmittel (31) mit dem Band (20) gleitbar gekoppelt sind.

3. Vorrichtung (1) nach einem der vorhergehenden Ansprüche, umfassend Spannmittel (31), welche mit einem der Enden des Bands (20) gekoppelt sind, und die Spannmittel (31) mit dem Band (20) gleitbar gekoppelt sind.

4. Vorrichtung (1) nach einem der vorhergehenden Ansprüche, wobei die Spannmittel (31) und die Regel-/Steuermittel (33) miteinander gekoppelt sind.

5. Vorrichtung (1) nach einem der Ansprüche 2 bis 4, wobei die Gleitkopplung durch Gleitmittel (35) erhalten wird, welche dem Maßband (20) zugeordnet sind, wobei die Gleitmittel (35) mit den Spannmitteln (32) gekoppelt sind.

6. Vorrichtung (1) nach dem vorhergehenden Anspruch, wobei die Spannmittel (31), welche mit den Gleitmitteln (35) gekoppelt sind, ferner mit den Regel-/Steuermitteln (33) gekoppelt sind.

7. Vorrichtung (1) nach Anspruch 5 oder 6, wobei die Gleitmittel (35) Einstellmittel umfassen, welche dazu eingerichtet sind, eine Gleitkopplung mit dem Maßband (20) zu erhalten.

8. Vorrichtung (1) nach dem vorhergehenden Anspruch, wobei die Einstellmittel wenigstens einen Durchgangsschlitz (38, 39) umfassen, durch welchen das Band (20) durchgeführt wird, und welcher einen Reibgleitabschnitt (40) mit dem Band (20) definiert.

9. Vorrichtung (1) nach einem der Ansprüche 5 bis 8, wobei die Einstellmittel zwei Durchgangsschlitze (38, 39) umfassen, welche dazwischen einen Reibgleitabschnitt (40) mit dem Band (20) definieren.

10. Vorrichtung (1) nach dem vorhergehenden Anspruch, wobei die Gleitkopplung durch Durchführen des Maßbands (20) durch den ersten Schlitz (38) von der ersten Fläche (20a) zu der zweiten Fläche (20b) des Bands (20) und durch den zweiten Schlitz (39) von der zweiten Fläche (20b) zu der ersten Fläche (20a) des Bands (20) rittlings des Reibgleitabschnitts (40) erhalten wird.

11. Vorrichtung (1) nach einem der vorhergehenden Ansprüche, wobei die Spannmittel (32) elastische Mittel sind.

12. Vorrichtung (1) nach dem vorhergehenden Anspruch, wobei die elastischen Mittel durch eine Schraubenfeder repräsentiert sind oder aus einem verformbaren Material gebildet sind, ausgewählt aus elastomeren Materialien.

13. Vorrichtung (1) nach einem der vorhergehenden Ansprüche, wobei die Regel-/Steuerelemente (34, 34a, 34b) in Bezug auf die Position der Anzeigeelemente (32) in dem Fall stationär sind, in dem keine Spannkraft auf die Spannmittel (32) angewandt wird.

14. Verfahren zum Messen des realen Umfangs einer Extremität in einem vorbestimmten Punkt, umfassend die Verwendung der Vorrichtung (1) nach einem der Ansprüche 1 bis 13, umfassend die Schritte:
1) Anwenden einer Spannkraft auf das Maßband (20) der Vorrichtung (1), wobei das Maßband (20) eine Extremität transversal gemäß dem Umfang in dem Punkt umgibt, durch die Spannmittel (31), um die vollständige Dehnung des Bands (20) und die vollständige Adhäsion an die Extremitätenoberfläche herzustellen;
2) Verfizieren der Anzeige des Umfangs in dem Punkt an der abgestuften Skala, während die Spannkraft von Schritt 1) konstant gehalten wird.

15. Verfahren zum Messen der Gewebekonsitenz in einem Punkt einer Extremität und zum Sammeln von zum Bewerten der fibrosklerotischen Änderung des Gewebes der Extremität nützlichen Informationen, umfassend die Verwendung der Vorrichtung (1) nach einem der Ansprüche 1 bis 13, umfassend die Schritte:
1) Anwenden einer Spannkraft auf das Maßband (20), wobei das Maßband (20) eine Extremität transversal gemäß dem Umfang in dem Punkt umgibt, durch die Spannmittel (31), um die vollständige Dehnung des Bands (20) und die vollständige Adhäsion an die Extremitätenoberfläche herzustellen;
2) Anwenden einer Vertiefungskraft durch die Spannmittel (31), um eine Vertiefung des Maßbands (20) auf das Hautgewebe zu erzeugen;
3) Verfizieren der verarbeiteten Anzeige des Extremitätenumfangs in dem Punkt an der abgestuften Skala und Verifizieren des Ausmaßes der Kraft eines Vertiefens, welche vermittels der Verifikation der Position der Regel-/Steuermittel (33) angewandt wird, während die Vertiefungskraft von Schritt 2) konstant gehalten wird.

## Revendications

1. Dispositif (1) pour mesurer la circonférence d'un membre, comprenant un mètre ruban (20) ayant deux faces (20a, 20b) et comprenant une échelle graduée et deux extrémités (21, 22), des moyens de mise sous tension (31) pour appliquer une force de tension (31) audit mètre ruban (20) capable de produire la distension totale de celui-ci, dans lequel les moyens de mise sous tension (31) comprennent des moyens d'indication (32) de ladite force de tension (31) quand ils sont soumis à ladite force de tension, et des moyens de contrôle (33) accouplés de manière fonctionnelle auxdits moyens de mise sous tension (31), lesdits moyens de contrôle (33) comprenant des éléments de contrôle (34, 34a, 34b) aptes à coopérer avec lesdits moyens d'indication (32) pour vérifier le déplacement des moyens d'indication (32) après l'application de ladite force de tension,
**caractérisé en ce que** lesdits moyens de mise sous tension (31) sont accouplés aux deux extrémités (21, 22) dudit mètre ruban (20).

2. Dispositif (1) selon la revendication précédente, dans lequel lesdits moyens de mise sous tension (31) sont accouplés de façon coulissante audit ruban (20).

3. Dispositif (1) selon l'une quelconque des revendications précédentes, comprenant des moyens de mise sous tension (31) accouplés aux extrémités dudit ruban (20), et des moyens de mise sous tension (31) accouplés de façon coulissante audit ruban (20).

4. Dispositif (1) selon l'une quelconque des revendications précédentes, dans lequel lesdits moyens de mise sous tension (31) et lesdits moyens de contrôle (33) sont mutuellement accouplés.

5. Dispositif (1) selon l'une quelconque des revendications 2 à 4, dans lequel ledit accouplement coulissant est obtenu par des moyens de coulissement (35) associés audit mètre ruban (20), lesdits moyens de coulissement (35) étant accouplés aux moyens de mise sous tension (32).

6. Dispositif (1) selon la revendication précédente, dans lequel lesdits moyens de mise sous tension (31) accouplés auxdits moyens de coulissement (35) sont accouplés en outre auxdits moyens de contrôle (33).

7. Dispositif (1) selon la revendication 5 ou 6, dans lequel lesdits moyens de coulissement (35) comprennent des moyens d'ajustement configurés pour obtenir un accouplement coulissant avec ledit mètre ruban (20).

8. Dispositif (1) selon la revendication précédente, dans lequel lesdits moyens d'ajustement comprennent au moins une fente de passage (38, 39) à travers laquelle le ruban (20) est passé, et définissant une partie de coulissement à frottement (40) avec ledit ruban (20).

9. Dispositif (1) selon l'une quelconque des revendications 5 à 8, dans lequel lesdits moyens d'ajustement comprennent deux fentes de passage (38, 39) définissant entre elles une partie de coulissement à frottement (40) avec le ruban (20).

10. Dispositif (1) selon la revendication précédente, dans lequel ledit accouplement coulissant est obtenu en passant ledit mètre ruban (20) à travers ladite première fente (38) à partir de la première face (20a) vers la seconde face (20b) du ruban (20) et à travers ladite seconde fente (39) à partir de la seconde face (20b) vers la première face (20a) du ruban (20) à cheval sur la partie de coulissement à frottement (40).

11. Dispositif (1) selon l'une quelconque des revendications précédentes, dans lequel lesdits moyens de mise sous tension (32) sont des moyens élastiques.

12. Dispositif (1) selon la revendication précédente, dans lequel lesdits moyens élastiques sont représentés par un ressort hélicoïdal ou sont faits d'un matériau déformable sélectionné parmi les matériaux élastomères.

13. Dispositif (1) selon l'une quelconque des revendications précédentes, dans lequel lesdits éléments de contrôle (34, 34a, 34b) sont fixes par rapport à la position des éléments d'indication (32) dans le cas où aucune force de tension n'est appliquée auxdits moyens de mise sous tension (32).

14. Procédé de mesure de la circonférence réelle d'un membre en un point prédéterminé, comprenant l'utilisation du dispositif (1) selon l'une quelconque des revendications 1 à 13, comprenant les étapes suivantes :
1) appliquer une force de tension au mètre ruban (20) du dispositif (1), ledit mètre ruban (20) entourant transversalement un membre selon la circonférence audit point, par l'intermédiaire des moyens de mise sous tension (31), de manière à produire la distension totale du ruban (20) et l'adhérence totale à la surface du membre ;
2) tandis que la force de tension de l'étape 1) est maintenue constante, vérifier l'indication de la circonférence audit point sur l'échelle graduée.

15. Procédé de mesure de la consistance d'un tissu en un point d'un membre et de collecte d'informations utiles pour évaluer l'altération fibroscléreuse des tissus dudit membre, comprenant l'utilisation du dispositif (1) selon l'une quelconque des revendications 1 à 13, comprenant les étapes suivantes :
1) appliquer une force de tension au mètre ruban (20), ledit mètre ruban (20) entourant transversalement un membre selon la circonférence audit point, par l'intermédiaire des moyens de mise sous tension (31), de manière à produire la distension totale du ruban (20) et l'adhérence totale à la surface du membre ;
2) appliquer une force d'approfondissement par l'intermédiaire des moyens de mise sous tension (31), de manière à produire un approfondissement du mètre ruban (20) sur le tissu cutané ;
3) tandis que la force d'approfondissement de l'étape 2) est maintenue constante, vérifier l'indication traitée de la circonférence du membre audit point sur l'échelle graduée et vérifier l'intensité de la force d'approfondissement appliquée en vertu de la vérification de la position des moyens de contrôle (33).
